# EUROPEAN PATENT APPLICATION

(11) **EP 2 752 666 A1**
(43) Date of publication of application: **09.07.2014**
(21) Application number: 12828352.0
(22) Date of filing: 28.08.2012
(51) Int. Cl.: G01N 33/68, C07K 16/18, C12Q 1/02, G01N 33/574

(54) **MARKER FOR DETECTING COLORECTAL CANCER OR ESOPHAGEAL CANCER AND METHOD FOR INSPECTING SAME**

(30) Priority: 29.08.2011 JP 2011186147
(71) Applicant: Toray Industries, Inc., Tokyo 103-8666 (JP); Kyoto University, Kyoto-shi, Kyoto 606-8501 (JP)
(72) Inventor: KOBAYASHI, Michimoto, Kamakura-shi Kanagawa 248-8555 (JP); TANAKA, Yoshinori, Kamakura-shi Kanagawa 248-8555 (JP); TAKAYAMA, Aiko, Kamakura-shi Kanagawa 248-8555 (JP); JUNG, Giman, Kamakura-shi Kanagawa 248-8555 (JP); SAKAI, Yoshiharu, Kyoto-shi Kyoto 606-8501 (JP); OKABE, Hiroshi, Kyoto-shi Kyoto 606-8501 (JP)
(74) Representative: Prüfer & Partner GbR European Patent Attorneys
(86) International application number: PCT/JP2012/071654
(87) International publication number: WO 2013/031756

(57) **Abstract**

This invention relates to a method for detecting or examining a cancer selected from colorectal cancer and esophageal cancer with high detection sensitivity and accuracy and with less invasiveness to a subject. Specifically, it relates to a method for examining a cancer comprising measuring *in vitro* a COTL1 protein in a body fluid sample from a subject and evaluating whether or not the subject suffers from the cancer based on the measured amount of the COTL1 protein. It further relates to a kit for diagnosis of a cancer selected from colorectal cancer and esophageal cancer comprising an antibody or a fragment thereof capable of specifically binding to the protein.

## Description

### Technical Field

The present invention relates to a method for examining colorectal cancer or esophageal cancer by measuring the concentration of a COTL1 protein as a marker for detecting colorectal cancer or esophageal cancer in a body fluid.

The present invention also relates to a kit for diagnosing colorectal cancer or esophageal cancer comprising a substance capable of binding to the protein used for detecting colorectal cancer or esophageal cancer.

### Background Art

Colorectal cancer is the second most common cancer, and patients with colorectal cancer account for 9.8% of the entire population of cancer patients. In 2008, worldwide, 17.3 people out of 100,000 developed colorectal cancer and 8.2 people out of 100,000 died therefrom. In Japan, colorectal cancer accounts for the second largest number of patients among cancers of any organs. As Western-style eating habits prevail, the number of patients with colorectal cancer is increasing in Japan.

In 2008, worldwide, 7.0 people out of 100,000 developed esophageal cancer and 5.8 people out of 100,000 died therefrom. The percentage of deaths worldwide caused by esophageal cancer is increasing year after year, and esophageal cancer accounted for 5.4% of all cancer deaths worldwide in 2008.

Treatment of colorectal cancer or esophageal cancer is performed via endoscopic therapy, surgery, chemotherapy, radiation therapy, or the like by taking disease stage, tumor size/depth, degree of metastasis, and other conditions into consideration. Early-stage cancer can be completely resected endoscopically or surgically, and the risk of recurrence is very low. In the case of advanced-stage cancer, however, metastases to the lungs, the liver, the lymph nodes, the peritoneum, or other regions that are difficult to resect occasionally occur, or local recurrence occasionally occurs at the site of resection. In such a case, radiation therapy and/or chemotherapy (anticancer drug therapy) are performed in addition to surgery. As described above, the prognosis of colorectal cancer and that of esophageal cancer are relatively favorable when found at relatively early stages, and 90% or more patients with early-stage cancer can be completely cured. However, the outcomes of large tumor or metastatic tumor cases are poor, and the importance of early detection is accordingly recognized.

Unfortunately, colorectal cancer and esophageal cancer are difficult to detect at an early stage based on subjective symptoms for the following reasons. That is, in most cases, colorectal cancer and esophageal cancer show substantially no symptoms at an early stage, and recognizable subjective symptoms are not developed until the cancer has become advanced. In the case of colorectal cancer, bowel-movement-related symptoms appear. In the case of esophageal cancer, symptoms such as discomfort when swallowing appear. However, such symptoms are often confused with those of other diseases, and such symptoms do not appear until the cancer grows to a certain extent. Accordingly, cancer that is diagnosed based on subjective symptoms has already undergone metastasis and has a poor prognosis in many cases.

Colorectal cancer is examined via digital rectal palpation, fecal occult blood test, or other means, and esophageal cancer is examined via esophagography, endoscopy, or other means. These techniques, however, are disadvantageous in terms of the necessity for long periods of time for definite diagnosis and the increased false-positive rate due to the difficulty of interpreting outcomes.

In order to detect colorectal cancer or esophageal cancer at the screening phase, discovery of a highly sensitive tumor marker in blood and examination performed with the use of such tumor marker are strongly demanded. The measurement of the level of such marker in blood is considered to allow relatively inexpensive high-throughput examination and diagnosis. To date, for example, CEA (Non-Patent Literature 1) and CA19-9 have been employed as markers for colorectal cancer, and SCC and CYFRA21-1 (Non-Patent Literature 2), as well as CEA, have been employed as markers for esophageal cancer in clinical settings.

### Prior Art Literatures

### Non-Patent Literature

Non-Patent Literature 1: Carpelan-Holmstrom, M. et al., 1995, British Journal of Cancer, Vol. 71, pp. 868-872
Non-Patent Literature 2: Quillien, V. et al., 1998, Oncology Report, Vol. 5, pp. 1,561-1,565

### Summary of Invention

### Problem to Be Solved by the Invention

The above-mentioned markers that have heretofore been employed in clinical settings, however, are poor in sensitivity for cancer detection. CEA, which is the most common marker for colorectal cancer, gives positive results for about 40% of colorectal cancer cases. CYFRA21-1, which is considered to exhibit the highest sensitivity for esophageal cancer, has sensitivity that is as low as about 40%. Accordingly, the use of such tumor markers is limited to post-treatment follow-up, and such tumor markers are not usually measured for the purpose of screening in general health checkups.

Accordingly, it is an object of the present invention to provide a novel tumor marker useful in detecting colorectal cancer or esophageal cancer and a method for detecting colorectal cancer or esophageal cancer using such tumor marker.

### Means for Solving the Problem

In order to attain the object, the present inventors have now compared protein groups present in the body fluids collected from colorectal cancer patients or esophageal cancer patients and the body fluids collected from healthy individuals to find the COTL1 protein as a novel tumor marker detected in body fluids collected from colorectal cancer or esophageal cancer patients. This has led to the completion of the present invention.

It has been reported that the "COTL1" (coactosin-like 1) protein, an actin cytoskeleton-binding protein, binds to 5-lipoxygenase in cells, and such protein has been considered to participate in leukotriene biosynthesis (Provost, P. et al., 2001, Journal of Biological Chemistry, Vol. 276, pp. 16,520-16,527). Also, it has been reported that the blood concentration of such protein increases by the onset of rheumatism (Eun-Heui, J. et al., 2009, Experimental and Molecular Medicine, Vol. 41, pp. 354-361). In addition, it has been known that the expression level of such protein is high in pancreatic cancer tissues (Nakatsura, T. et al., 2001, Biochemical and Biophysical Research Communication, Vol. 256, pp. 75-80). However, there have been no reports concerning the correlation between the COTL1 protein and colorectal cancer or esophageal cancer.

Thus, the present invention encompasses the following features.
(1) A method for examining a cancer comprising measuring *in vitro* an amount of a cancer detecting marker consisting of a COTL1 protein present in a body fluid from a subject, and evaluating whether or not the subject suffers from a cancer selected from colorectal cancer and esophageal cancer on the basis of the amount of the marker.
(2) The method according to (1), wherein the COTL1 protein is a polypeptide consisting of the amino acid sequence as shown in SEQ ID NO: 1, an amino acid sequence having 90% or higher identity with the amino acid sequence of SEQ ID NO: 1, or a partial sequence comprising at least 7-10 or more continuous amino acid residues constituting each of the amino acid sequences.
(3) The method according to (1) or (2), wherein, when the amount of the cancer detecting marker in the subject is statistically significantly larger than that of a healthy individual, the subject is evaluated or determined as suffering a cancer selected from colorectal cancer and esophageal cancer.
(4) The method according to (3), wherein the statistically significantly larger amount is two or more times that of a healthy individual.
(5) The method according to any one of (1) to (4), wherein the measurement is performed using a substance capable of specifically binding to the cancer detecting marker.
(6) The method according to (5), wherein the substance capable of binding is an anti-COTL1 antibody and/or a fragment thereof.
(7) The method according to any one of (1) to (6), wherein the body fluid sample is blood or urine.
(8) A kit for diagnosing a cancer selected from colorectal cancer and esophageal cancer, comprising an anti-COTL1 antibody, a fragment thereof, and/or a chemically modified derivative thereof.

The present specification encompasses the contents described in the specification and/or drawings of Japanese Patent Application No. 2011-186147, from which the present application claims priority.

According to the present invention, the cancer selected from colorectal cancer or esophageal cancer can be detected with higher sensitivity than that attained with conventional tumor markers. By measuring the concentration of the COTL1 protein contained in a body fluid sample such as the blood of a patient suspected of having colorectal cancer or esophageal cancer, for example, whether or not the patient has colorectal cancer or esophageal cancer can be diagnosed or evaluated.

### Brief Description of Drawings

[Fig. 1] This figure is a chart showing the results of measuring the COTL1 protein in the plasmas of healthy persons and colorectal cancer patients by Western blotting.
[Fig. 2] This figure is a chart showing the results of measuring the COTL1 protein in the plasmas of healthy persons and esophageal cancer patients by Western blotting. Embodiments of the Invention

### 1. Cancer detecting marker

The first aspect of the present invention relates to a cancer detecting marker that is intended for use in evaluating whether or not a subject suffers from a cancer selected from colorectal cancer or esophageal cancer. The present invention is based on the findings that the COTL1 protein is more abundant in body fluids, such as bloods, of colorectal cancer or esophageal cancer patients than in those of healthy persons. As described in the second aspect of the present invention below, colorectal cancer or esophageal cancer affecting a subject can be detected based on an increased amount of this protein present in a body fluid, such as blood, of a subject.

In the present invention, the "cancer detecting marker" is a biological marker intended for use in detecting colorectal cancer or esophageal cancer, and it refers to a substance that serves as an indicator showing that the subject has colorectal cancer or esophageal cancer. The COTL1 protein constitutes the cancer detecting marker of the present invention. The term "COTL1 protein" used herein refers not only to a full-length COTL1 protein that is predominantly present in a vertebrate (preferably a mammal) but also to a variant thereof and/or a fragment thereof. These are collectively referred to as "COTL1 protein" herein.

The "COTL1 protein" of the present invention is an actin cytoskeleton-binding protein, as described above. In the present invention, the COTL1 protein corresponds to, for example, an approximately 17 kDa mammal-derived COTL1 protein composed of approximately 142 amino acids, and it is preferably a human-derived COTL1 protein (GenBank Accession No. NP_066972.1). Specifically, it is a polypeptide comprising the amino acid sequence as shown in SEQ ID NO: 1. Also, the COTL1 protein may be a full-length COTL1 protein that is majorly present in a human, a human-derived variant thereof, and/or a human-derived fragment thereof. The present inventors have revealed that the COTL1 protein is produced by colorectal cancer or esophageal cancer cells and leaked out in larger amounts into the body fluids of colorectal cancer or esophageal cancer patients than into those of healthy individuals, by a statistically significant degree.

The "variant" of the COTL1 protein used herein refers to a variant comprising an amino acid sequence derived from an amino acid sequence constituting the COTL1 protein (preferably the human-derived COTL1 protein, as shown in SEQ ID NO: 1) or a partial sequence thereof by deletion, substitution, addition, or insertion of one or more, and preferably one to several, amino acids, or a variant exhibiting approximately 80% or higher, approximately 85% or higher, preferably approximately 90% or higher, more preferably approximately 95% or higher, approximately 97% or higher, approximately 98% or higher, or approximately 99% or higher percent identity to the aforementioned amino acid sequence or a partial sequence thereof. In this context, the term "several" refers to an integer that is approximately 10, 9, 8, 7, 6, 5, 4, 3, or 2 or smaller. The "% identity" can be determined with or without the introduction of a gap using a BLAST- or FASTA-based protein search system (Karlin, S. et al., 1993, Proceedings of the National Academic Sciences, U.S.A., Vol. 90, pp. 5,873-5,877; Altschul, S.F. et al., 1990, Journal of Molecular Biology, Vol. 215, pp. 403-410; and Pearson, W. R. et al., 1988, Proceedings of the National Academic Sciences, U.S.A., Vol. 85, pp. 2,444-2,448). Specific examples of the variant of the COTL1 protein include variants having a polymorphism (including SNIPs) based on the type of subject (e.g., the race of a human subject) or individual, and splicing variants.

The term "fragment" of the COTL1 protein used herein refers to a polypeptide fragment comprising at least 7-10 to less than all, at least 15 to less than all, preferably at least 20 to less than all, at least 25 to less than all, and more preferably at least 35 to less than all, at least 40 to less than all, or at least 50 to less than all continuous amino acid residues constituting the COTL1 protein (preferably the human-derived COTL1 protein, as shown in SEQ ID NO: 1) or a variant thereof, and such polypeptide fragment retains one or more epitopes. Such a fragment can immunospecifically bind to the antibody according to the present invention described below or a fragment thereof. Such peptide fragment is within the scope of the COTL1 protein for the following reasons: the object of the present invention can be attained, as long as the COTL1 protein, even if fragmented, in blood can be quantified; and the full-length polypeptide of the COTL1 protein (preferably the human-derived COTL1 protein of SEQ ID NO: 1 or a variant thereof) in blood may be found to be fragmented by, for example, proteases, peptidases or the like present in the blood.

### 2. Method for examining colorectal cancer or esophageal cancer

The second aspect of the present invention relates to a method for detecting or examining *in vitro* a cancer selected from colorectal cancer or esophageal cancer.

The term "cancer" used herein is intended to encompass a malignant tumor and a carcinoma. The cancer may be either invasive or noninvasive, and it may be either a primary cancer or a metastatic cancer.

On the basis of findings to the effect that the COTL1 protein is more abundant in a body fluid, such as blood, of a colorectal cancer or esophageal cancer patient than in that of a healthy person, the present invention provides a method of measuring the amount of the cancer detecting marker of the invention present in a body fluid derived from a subject and evaluating whether or not the subject suffers from colorectal cancer or esophageal cancer based on the results of measurement.

The method of the present invention comprises (1) a step of measuring the cancer detecting marker and (2) a step of determining suffering from the cancer. Hereinafter, each step will be described in detail.

### 2-1. Step of measuring the cancer detecting marker

The "step of measuring the cancer detecting marker" is a step of measuring *in vitro* the amount of the cancer detecting marker of the present invention, i.e., the COTL1 protein, that is present in a body fluid derived from a subject.

The term "subject" used herein refers to a subject subjected to detection of suffering from colorectal cancer or esophageal cancer, and it is a vertebrate, preferably a mammal, particularly preferably a human. Hereinafter, a human serving as a subject is specifically referred to as a "human subject."

The term "body fluid" used herein refers to a sample subjected to detection of colorectal cancer or esophageal cancer, and it is a biological fluid material. The body fluid is not particularly limited, and it may be any biological fluid material possibly containing the cancer detecting marker of the present invention. Examples thereof include blood, urine, culture supernatants of lymphocytes, spinal fluid, digestive juice (including intestinal juice, secretion from the esophageal glands, and saliva), sweat, ascites fluid, runny nose fluid, tears, vaginal fluid, and seminal fluid, with blood or urine being preferable. The term "blood" used herein refers to whole blood, plasma, or serum. Whole blood may be venous blood, arterial blood, or cord blood. The body fluid may be a combination of two or more different body fluids obtained from one individual. Since the method for detecting colorectal cancer or esophageal cancer of the present invention can be carried out with the use of blood or urine in a less invasive manner, such method is very useful for detection from the viewpoint of convenience.

The term "body fluid from a subject" used herein refers to a body fluid that has already been collected from a subject. The act itself of collecting the body fluid is not encompassed by this aspect of the present invention. The body fluid from a subject may be subjected to the method of the present invention immediately after being collected from the subject. Alternatively, the collected body fluid may be subjected to refrigeration or freezing or appropriate treatment followed by refrigeration or freezing, and before subjected to the method of the present invention, the refrigerated or frozen body fluid may be raised to room temperature. Examples of appropriate treatment before refrigeration or freezing include addition of heparin or the like to whole blood for anticoagulation treatment, followed by separation of plasma or serum. Such treatment can be performed on the basis of a technique known in the art.

The term "amount of the cancer detecting marker of the present invention" refers to the quantity of the COTL1 protein present in a body fluid from a subject. This quantity may be either an absolute amount or a relative amount. The absolute amount corresponds to the mass or volume of the cancer detecting marker contained in a predetermined amount of a body fluid. The relative amount is indicated in the form of the measured value of the subject-derived marker for detecting cancer relative to a particular measured value. Examples thereof include concentration, fluorescence intensity, and absorbance.

The amount of the cancer detecting marker can be measured *in vitro* using a method known in the art. An example thereof is a measurement method using a substance capable of specifically binding to the protein.

The expression "capable of specifically binding" used herein means that a certain substance forms a complex substantially only with the cancer detecting marker, i.e., the COTL1 protein, which is the target of the present invention. In this context, the term "substantially" refers to a situation in which a complex may be formed via unspecific binding at an insignificant level, to such an extent that the method of the present invention is not affected.

Examples of the "substance capable of specifically binding" include COTL1-binding proteins. More specifically, the substance capable of specifically binding is, for example, an "anti-COTL1 antibody" recognizing and binding to the COTL1 protein as an antigen, and preferably an antibody recognizing and binding to the polypeptide comprising the amino acid sequence as shown in SEQ ID NO: 1 or an antibody recognizing and binding to a variant of the above-mentioned polypeptide as an antigen; that is, an antibody recognizing and binding to a polypeptide having an amino acid sequence that is a variant of the sequence of SEQ ID NO: 1 and/or an antibody fragment thereof. Alternatively, the substance capable of specifically binding may be a chemically modified derivative thereof. In this context, the term "chemically modified derivative" encompasses any functional modifications necessary for acquiring or retaining the specific binding activity of the anti-COTL1 antibody or a fragment thereof and any modifications for labeling necessary for detecting the anti-COTL1 antibody or a fragment thereof.

Examples of functional modifications include glycosylation, deglycosylation, and PEGylation.

Examples of labeling modifications include labeling with a fluorescent dye (FITC, rhodamine, Texas Red, Cy3, or Cy5), a fluorescent protein (e.g., PE, APC, and GFP), an enzyme (e.g., horseradish peroxidase, alkaline phosphatase, and glucose oxidase), biotin, avidin, and streptavidin.

The antibody may be a polyclonal or monoclonal antibody. The monoclonal antibody is preferable for realization of specific detection. The anti-COTL1 polyclonal or monoclonal antibody specifically binding to the COTL1 protein can be prepared by methods described later. In addition, an anti-human COTL1 polyclonal antibody is commercially available from Protein Group Inc., etc., and it may be used in the present invention. The globulin type of the antibody of the present invention is not particularly limited, as long as it has the features described above. The globulin type may be IgG, IgM, IgA, IgE, or IgD, with IgG and IgM being preferable. Examples of the antibody fragment include, but are not limited to, Fab, Fab', F(ab')₂, Fv, and ScFv. The antibody of the present invention also encompasses an antibody fragment and a derivative that can be produced by a genetic engineering technique. Examples of such antibody include synthetic antibody, recombinant antibody, multispecific antibody (including bispecific antibody), and single-chain antibody. The anti-COTL1 protein antibody of the present invention or fragment thereof is an antibody against one or several epitopes each comprising at least 5, and preferably at least 7-10 or 8-10 continuous or discontinuous amino acid residues of the protein. The specific polyclonal antibody can be prepared by, for example, a technique comprising applying the antiserum of a rabbit or the like immunized with the protein to a column comprising the COTL1 protein (e.g., a protein surface antigen (poly)peptide) conjugated with a carrier such as agarose, and collecting the IgG antibody bound to the column carrier.

### (1) Preparation of anti-COTL1 antibody

Hereafter, methods for preparing the anti-COTL1 polyclonal antibody and the monoclonal antibody used in the present invention are described in detail.

### (1-1) Preparation of immunogen

In the present invention, a COTL1 protein is first prepared as an immunogen (antigen) in order to prepare the antibody. A COTL1 protein that can be used as an immunogen in the present invention is, for example, a human COTL1 protein comprising the amino acid sequence as shown in SEQ ID NO: 1, a variant thereof, a polypeptide fragment thereof, or a fusion polypeptide of any thereof with another peptide (e.g., a signal peptide or a labeling peptide). For example, a COTL1 protein fragment to be used as an immunogen can be synthesized by a technique known in the art, such as solid-phase peptide synthesis, using information concerning the amino acid sequence of SEQ ID NO: 1. A COTL1 protein fragment serving as an immunogen is preferably conjugated to a carrier protein, such as KLH or BSA.

Also, a COTL1 protein as an immunogen can be obtained using a DNA recombination technique known in the art. cDNA encoding the COTL1 protein can be prepared by a cDNA cloning method. Total RNA is extracted from biological tissues such as epithelial cells expressing the gene of immunogenic COTL1 and treated with an oligo-dT cellulose column. A cDNA library can be prepared by RT-PCR using the obtained poly-A(+) RNA as a template, and the resulting cDNA library can be subjected to hybridization screening, expression screening, antibody screening, or other means, so as to obtain the cDNA clone of interest. The cDNA clone may be further amplified by PCR, according to need. As a result, cDNA corresponding to the gene of interest can be obtained. Such cDNA cloning technique is described in, for example, Sambrook, J. and Russell, D., Molecular Cloning, A Laboratory Manual, Cold Spring Harbor Laboratory Press, issued on January 15, 2001, Vol. 1: 7.42 to 7.45 and Vol. 2: 8.9 to 8.17.

Subsequently, the cDNA clone thus obtained is incorporated into expression vectors, prokaryotic or eukaryotic host cells are transformed or transfected using such vectors, and the resulting cells can be cultured to obtain the COTL1 protein of interest therefrom. When the protein of interest is obtained from the culture supernatant thereof, a nucleotide sequence encoding a secretory signal sequence can be flanked by the 5' end of DNA encoding the polypeptide to thereby extracellularly secrete a mature polypeptide.

Examples of the expression vectors include *E. coli*-derived plasmids (e.g., pET21a, pGEX4T, pC118, pC119, pC18, and pC19), *Bacillus subtilis*-derived plasmids (e.g., pUB110 and pTP5), yeast-derived plasmids (e.g., YEp13, YEp24, and YCp50), and phage DNA such as λ phages (e.g., λgt11 and λZAP). In addition, an animal virus such as vaccinia virus or an insect virus vector such as baculovirus may be used. Such vectors and expression systems are available from, for example, Novagen, Takara Shuzo Co., Ltd., Daiichi Pure Chemicals Co., Ltd., Qiagen, Stratagene, Promega Corp., Roche Diagnostics, Life Technologies, Genetics Institute, Inc., and GE Healthcare.

For example, a method involving cleaving purified DNA with appropriate restriction enzymes and inserting the resulting fragment into an appropriate restriction enzyme site or a multicloning site to ligate the fragment to the vector is adopted for inserting DNA (e.g., cDNA) encoding the COTL1 protein into an expression vector. The vector can contain, in addition to the DNA encoding the protein, regulatory elements, such as a promoter, an enhancer, a polyadenylation signal, a ribosome-binding site, a replication origin, a terminator, and a selection marker. Alternatively, a labeling peptide may be applied to the C- or N-terminus of a polypeptide, and the resulting fusion polypeptide may be used to facilitate polypeptide purification. Typical examples of the labeling peptide include, but are not limited to, a histidine repeat of 6-10 residues, FLAG, myc peptide, and GFP protein. The DNA recombination technique is described in Sambrook, J. & Russell, D. (described above). A DNA fragment is ligated to a vector fragment using DNA ligase known in the art.

Prokaryotic cells such as bacteria (e.g., *Escherichia coli* and *Bacillus subtilis*), yeast (e.g., *Saccharomyces cerevisiae*), insect cells (e.g., Sf cells), mammalian cells (e.g., COS, CHO, and BHK), or the like can be used as host cells. Methods for introducing the recombinant vectors into host cells are not particularly limited, as long as they allow DNA to be introduced into a relevant host. Examples of methods for introducing the vectors into bacteria include a heat shock method, a method using calcium ions, and electroporation. These techniques are known in the art and described in various documents (see, for example, Sambrook, J. & Russell, D. (as above)). Preferably, the vectors can be introduced into animal cells by, for example, a lipofection method (PNAS, 1989, Vol. 86, 6077; and PNAS, 1987, Vol. 84, 7413), electroporation, a calcium phosphate method (Virology, 1973, Vol. 52, 456-467), a method using liposomes, or a DEAE-dextran method.

Either a natural or synthetic medium may be used for the culture of transformants obtained with a microorganism (such as *E. coli* or yeast) as hosts, as long as such medium contains a carbon source, a nitrogen source, and inorganic salts assimilable by the microorganisms, and as long as the transformants can be efficiently cultured therein. The culture is generally performed at 37°C for 6 to 24 hours under aerobic conditions such as shake culture or aeration and agitation culture. During the culture period, the pH is kept at around neutral. The pH is adjusted using an inorganic or organic acid, an alkaline solution, or the like. An antibiotic such as ampicillin or tetracycline may be added to the medium, if necessary, during the culture. Transformants such as mammalian cells are also cultured in a medium suitable for each type of cells, and proteins produced in the culture supernatant or the cells are then collected. In this procedure, the medium may or may not contain serum, although culture conducted in a serum-free medium is preferable. When the COTL1 protein is produced within bacteria or cells, these bacteria or cells are disrupted to extract the protein. Alternatively, when the COTL1 protein is produced outside the bacteria or cells, the culture solution is used in that state, or the bacteria or cells are removed therefrom by centrifugation or other means.

When the protein according to the present invention is produced in a form that is not tagged with a labeling peptide, the protein can be purified by, for example, a method based on ion-exchange chromatography. This method may be performed in combination with, for example, gel filtration, hydrophobic chromatography, or isoelectric chromatography. When the protein is tagged with a labeling peptide such as a histidine repeat, FLAG, myc, or GFP, in contrast, a common method based on affinity chromatography suitable for each labeling peptide can be employed. Whether or not the COTL1 protein is obtained can be confirmed by SDS-polyacrylamide gel electrophoresis or other means.

### (1-2) Preparation of antibody

The COTL1 protein thus obtained can be used as an antigen to obtain an antibody specifically recognizing the COTL1 protein.

More specifically, proteins, protein fragments, protein variants, fusion proteins, and the like contain antigenic determinants or epitopes that induce antibody formation. These antigenic determinants or epitopes may be linear (continuous) or have a higher order structure (discontinuous). The antigenic determinants or epitopes can be identified by any method known in the art.

The COTL1 protein of the present invention can induce antibodies in a variety of forms. A polyclonal or monoclonal antibody can be prepared via a conventional technique, as long as the whole of or a portion of the protein or its epitope is isolated. An example of such technique is the method described in Kennet et al. (ed.), Monoclonal Antibodies, Hybridomas: A New Dimension in Biological Analyses, Plenum Press, New York, 1980.

### (1-2-1) Preparation of polyclonal antibody

In order to prepare the polyclonal antibody, the obtained COTL1 protein is first dissolved in a buffer to prepare an immunogen. An adjuvant may be added, if necessary, for effective immunization. Examples of adjuvant include commercially available Freund's complete adjuvant (FCA) and Freund's incomplete adjuvant (FIA). These adjuvants can be used alone or as a mixture.

Next, the immunogen thus prepared is administered to mammals such as rats, mice (e.g. Balb/c mice of an inbred line), or rabbits for immunization. A single dose of the immunogen is adequately determined in accordance with the type of animal to be immunized, the route of administration, and other conditions, and it may be approximately 50 to 200 µg per animal. Examples of methods for administering the immunogen include, but are not limited to, hypodermic injection using FIA or FCA, intraperitoneal injection using FIA, and intravenous injection using 0.15 mol/l sodium chloride. The immunization interval is not particularly limited. After the initial immunization, 2 to 10, and preferably 3 to 4, boosters are performed at several-day to several-week intervals, and preferably 1- to 4-week intervals. After the initial immunization, an antibody titer in the serum of the immunized animals is repetitively measured by ELISA (enzyme-linked immunosorbent assay) or other means. When the antibody titer reaches a plateau, the immunogen is intravenously or intraperitoneally injected into the animals for final immunization. Thereafter, a polyclonal antibody against the COTL1 protein can be collected from the blood. If a monoclonal antibody is required, the anti-COTL1 antibody-producing hybridomas described below may be prepared.

### (1-2-2) Preparation of monoclonal antibody

According to the present invention, hybridomas producing an anti-COTL1 monoclonal antibody specifically recognizing the COTL1 protein can be prepared. Such hybridomas can be produced and identified by a conventional technique. For example, a method for producing such hybridomas can involve: immunizing animals with the protein of the present invention; collecting antibody-producing cells from the immunized animals; fusing the antibody-producing cells to a myeloma cell line to thereby form hybridoma cells; and identifying hybridomas producing the monoclonal antibody binding to the COTL1 protein.

### <Collection of antibody-producing cell from immunized animal>

Examples of the antibody-producing cells include spleen cells, lymph node cells, and peripheral blood cells, preferably spleen cells or local lymph node cells. These cells can be used after being extracted or collected from the animals immunized with the COTL1 protein. The method for immunizing animals is in accordance with the "Preparation of polyclonal antibody" section above. Generally available established cell lines from animals such as mice can be used as the myeloma cell lines to be fused with the antibody-producing cells. It is preferable that the cell lines have drug-selectivity and be unable to survive in a HAT selection medium (containing hypoxanthine, aminopterin, and thymidine), unless they are fused to antibody-producing cells. It is also preferable that the established cell line be derived from an animal of the same line as the immunized animal. Specific examples of the myeloma cell lines include BALB/c mouse-derived hypoxanthine-guanine phosphoribosyltransferase (HGPRT)-deficient cell lines, such as P3X63-Ag.8 (ATCC TIB9), P3X63-Ag.8.U1 (JCRB9085), P3/NSI/1-Ag4-1 (JCRB0009), P3x63Ag8.653 (JCRB0028), and Sp2/0-Ag14 (JCRB0029).

### <Cell fusion>

For cell fusion, the antibody-producing cells are mixed with the myeloma cell line at a ratio of approximately 1:1 to 20:1 in a medium for animal cell culture, such as a serum-free DMEM or RPMI-1640 medium, and subjected to a fusion reaction in the presence of a cell fusion promoter. For example, polyethylene glycol having an average molecular weight of 1,500 to 4,000 daltons (Da) can be used as the cell fusion promoter at a concentration of approximately 10% to 80%. In some cases, the cell fusion promoter may be used in combination with an auxiliary agent such as dimethyl sulfoxide for enhanced fusion efficiency. Further, the antibody-producing cells may be fused with the myeloma cell line using a commercially available cell fusion apparatus based on electric stimulation (e.g., electroporation) (Nature, 1977, Vol. 266, 550-552).

### <Selection and cloning of hybridomas>

Hybridomas producing the anti-COTL1 antibody of interest are selected from among the cells after the cell fusion treatment. A method therefor involves: appropriately diluting the cell suspension with, for example, a fetal bovine serum-containing RPMI-1640 medium; seeding the resultant at a density of approximately 2,000,000 cells/well onto a microtiter plate; adding a selection medium to each well; and conducting culture while appropriately exchanging selection media. The culture temperature is 20°C to 40°C, and preferably approximately 37°C. When the myeloma cells are of the HGPRT-deficient or thymidine kinase-deficient line, hybridomas from the cells having the ability to produce antibodies and the myeloma cell line can be selectively cultured and grown using a selection medium containing hypoxanthine, aminopterin, and thymidine (HAT medium). As a result, the grown cells can be obtained as hybridomas approximately 14 days after the initiation of culture in the selection medium.

Next, the culture supernatant of the grown hybridomas is screened to confirm the presence or absence of the antibody of interest. Screening of hybridomas can be performed in accordance with a conventional technique without particular limitation. For example, a portion of the culture supernatant in each well containing the grown hybridomas can be collected and screened by enzyme immunoassay (EIA, and ELISA) or radioimmunoassay (RIA). The fusion cells are cloned by a limiting dilution method or other means. In the end, hybridomas are established as monoclonal antibody-producing cells. The hybridomas of the present invention are stable during culture in a basal medium such as RPMI-1640 or DMEM, as described below, and produce or secrete a monoclonal antibody specifically reacting with the colorectal cancer or esophageal cancer-derived COTL1 protein.

### <Collection of antibody>

The monoclonal antibody can be collected by a conventional technique. Specifically, a general cell culture or ascites fluid formation technique can be adopted for collecting the monoclonal antibody from the established hybridomas. In the cell culture technique, the hybridomas are cultured for 2 to 10 days under general culture conditions (e.g., 37°C, 5% CO₂ concentration) in a medium for animal cell culture such as a RPMI-1640 or MEM medium containing 10% fetal bovine serum or a serum-free medium, and the antibody is obtained from the culture supernatant. In the case of ascites fluid formation, approximately 10,000,000 hybridomas are intraperitoneally administered to an animal of the same line as the mammal from which the myeloma cells are derived, so that large quantities of hybridomas can grow. Ascites fluid or serum is collected 1 to 2 weeks thereafter.

When the method for collecting the antibody requires antibody purification, the purified monoclonal antibody of the present invention can be obtained by appropriately selecting or combining method(s) known in the art, such as salting out with ammonium sulfate, ion-exchange chromatography, affinity chromatography, and gel chromatography.

The monoclonal antibody of the present invention encompasses a chimeric antibody, such as a humanized form of a murine monoclonal antibody. The present invention also provides an antigen-binding fragment of the antibody. Examples of the antigen-binding fragment that can be produced by a conventional technique include, but are not limited to, Fab, F(ab')₂, and Fv fragments. The present invention also provides an antibody fragment and a derivative that can be produced by a genetic engineering technique. The antibody of the present invention can be used in assay for detecting the presence of the polypeptide of the present invention or the (poly)peptide fragment thereof *in vitro* and *in vivo*. Moreover, the antibody of the present invention can be used in the purification of a protein or a protein fragment by immunoaffinity chromatography.

In order to realize specific detection in assays, use of the monoclonal antibody is preferable. Even in the case of the polyclonal antibody, specific antibodies can be obtained by a so-called absorption method involving binding antibodies to an affinity column conjugated with purified polypeptides.

### (2) In vitro measurement of cancer detecting marker of the present invention using anti-COTL1 antibody or the like

Examples of methods for measuring *in vitro* the amount of the cancer detecting marker of the present invention, i.e., the COTL1 protein, present in a body fluid derived from a human subject using the anti-COTL1 antibody prepared in (1) above (immunological assay methods) include enzyme immunoassay (ELISA and EIA), fluorescent immunoassay, radioimmunoassay (RIA), luminescent immunoassay, immunonephelometry, latex agglutination reaction, latex turbidimetry, hemagglutination reaction, particle agglutination reaction, and Western blotting.

When the method for measuring the cancer detecting marker of the present invention is carried out via immunoassay using a label (via, for example, enzyme immunoassay, fluorescent immunoassay, radioimmunoassay, or luminescent immunoassay), it is preferable that the anti-COTL1 antibody or components in the sample be immobilized onto a solid phase and allowed to undergo immunological reactions. An insoluble carrier in the form of, for example, beads, a microplate, a test tube, a stick, or a test piece made of a material such as polystyrene, polycarbonate, polyvinyl toluene, polypropylene, polyethylene, polyvinyl chloride, nylon, polymethacrylate, latex, gelatin, agarose, cellulose, Sepharose, glass, metal, ceramics, or a magnetic substance can be used as a solid phase carrier. Immobilization can be performed by binding the anti-COTL1 antibody or sample components to the solid phase carrier in accordance with a method known in the art, such as physical adsorption or chemical binding, or a combination thereof.

In the present invention, the reaction of the anti-COTL1 antibody with the cancer detecting marker of the present invention derived from colorectal cancer or esophageal cancer cells in the body fluid can be easily detected either directly by labeling the anti-COTL1 antibody or indirectly by using a labeled secondary antibody. According to the method for detecting cancer of the present invention, the latter indirect method (e.g., a sandwich method) is preferable from the viewpoint of sensitivity.

A labeling material such as peroxidase (POD), alkaline phosphatase, β-galactosidase, urease, catalase, glucose oxidase, lactate dehydrogenase, amylase, or a biotin-avidin complex can be used for enzyme immunoassay; a labeling material such as fluorescein isothiocyanate, tetramethylrhodamine isothiocyanate, substituted rhodamine isothiocyanate, dichlorotriazine isothiocyanate, Alexa, or Alexa Fluoro can be used for fluorescent immunoassay; and a labeling material such as tritium, iodine 125, or iodine 131 can be used for radioimmunoassay. Alternatively, a labeling material such as NADH-, FMNH2-, luciferase system, luminol-hydrogen peroxide-POD system, acridinium ester system, or dioxetane compound system can be used for luminescent immunoassay.

In the case of enzyme immunoassays, a labeling material can be bound to an antibody by a method known in the art, such as a glutaraldehyde, maleimide, pyridyl disulfide, or periodic acid method. In the case of radioimmunoassay, a conventional technique, such as a chloramine-T method or Bolton-Hunter method, can be employed. Such assays can be carried out in accordance with a conventional technique (Current protocols in Protein Sciences, 1995, John Wiley & Sons, Inc.; and Current protocols in Immunology, 2001, John Wiley & Sons, Inc.).

When the anti-COTL1 antibody is directly labeled, for example, components in the body fluid are immobilized on a solid phase and brought into contact with the labeled anti-COTL1 antibody to form a complex of the cancer detecting marker (the COTL1 protein) of the present invention with the anti-COTL1 antibody. The labeled antibodies that are not bound are separated by washing, and the amount of the cancer detecting marker (the COTL1 protein) in the body fluid can be determined on the basis of the amount of the labeled antibody that is bound or the labeled antibody that is not bound.

Alternatively, when a labeled secondary antibody is used, for example, the antibody of the present invention is allowed to react with the sample (i.e., the primary reaction) and is then allowed to react with a labeled secondary antibody (i.e., the secondary reaction). The primary reaction and the secondary reaction may be performed in reverse order, simultaneously, or at different times. As a result of the primary reaction and the secondary reaction, a complex of the immobilized cancer detecting marker of the present invention, the anti-COTL1 antibody, and the labeled secondary antibody, or a complex of the immobilized anti-COTL1 antibody, the cancer detecting marker of the present invention, and the labeled secondary antibody is formed. The labeled secondary antibody that is not bound is then separated by washing, and the mass of the cancer detecting marker in the sample can be determined on the basis of the amount of the labeled secondary antibody that is bound or the labeled secondary antibody that is not bound.

In the case of enzyme immunoassay, specifically, the labeling enzyme is allowed to react with a substrate under the optimal conditions, and the amount of the reaction product is measured by an optical method or the like. Alternatively, fluorescence intensity derived from labeling with a fluorescent material or radioactivity derived from labeling with a radioactive substance is measured for fluorescent immunoassay and radioimmunoassay, respectively. For luminescent immunoassay, the amount of luminescence from the luminescence reaction system is measured.

In the method of the present invention, the formation of agglutinated immune complexes through immunonephelometry, latex agglutination reaction, latex turbidimetry, hemagglutination reaction, particle agglutination reaction, or the like can be determined via optical assay of transmitted or scattered light thereof or via visual observation using, for example, a phosphate buffer, a glycine buffer, a Tris buffer, or a Good's buffer as a solvent. The reaction system may further contain a reaction promoter such as polyethylene glycol or a nonspecific reaction inhibitor.

A preferable embodiment of the detection method of the present invention is described below. At the outset, the antibody of the present invention is immobilized as a primary antibody onto an insoluble carrier. Preferably, the surface of the solid phase to which the antigen is not adsorbed is subjected to blocking with a protein (e.g., calf serum, bovine serum albumin, or gelatin) irrelevant to the antigen. Subsequently, the immobilized primary antibody is brought into contact with a test sample. A labeled secondary antibody that reacts with the cancer detecting marker of the present invention is brought into contact therewith at a site different from that of the primary antibody, and a signal from the label is then detected. In this context, the "secondary antibody that reacts with the cancer detecting marker at a site different from that of the primary antibody" is not particularly limited, as long as this antibody recognizes a site other than the binding site between the primary antibody and the cancer detecting marker (the COTL1 protein). A polyclonal antibody, antiserum, or a monoclonal antibody may be used, irrespective of the type of the immunogen. Alternatively, an antibody fragment (e.g., Fab, F(ab')₂, Fab, Fv, or ScFv) thereof may be used. Moreover, several types of monoclonal antibodies may be used as such secondary antibodies.

Alternatively, the antibody of the present invention may be labeled and used as a secondary antibody. In such case, the antibody that reacts with the cancer detecting marker at a site different from that of the antibody of the present invention is immobilized as a primary antibody onto an insoluble carrier, and this immobilized primary antibody is brought into contact with a test sample and then with the labeled antibody of the present invention as a secondary antibody. A signal from the label may then be detected.

As described above, the antibody of the invention reacts specifically with the cancer detecting marker derived from colorectal cancer or esophageal cancer cells. Thus, it can be used as an agent for detecting cancer. Since the agent for detecting cancer of the invention comprises the antibody of the invention, the agent for detecting cancer of the invention may be used to detect a cancer detecting marker, which is derived from the colorectal cancer or esophageal cancer cells, contained in a sample collected from an individual suspected of colorectal cancer or esophageal cancer. This agent can detect or examine the presence or absence of colorectal cancer or esophageal cancer in an individual or whether or not the individual suffers from such cancer.

Also, the agent for detecting cancer of the invention can be used for any immunological assay means. The agent for detecting cancer of the invention can be used in combination with convenient means known in the art, such as a test strip for immunochromatography, to thereby detect cancer more easily and rapidly. The test strip for immunochromatography may comprise, for example: a sample-receiving section made of a material easily absorbing a sample; a reagent section containing the agent for detecting cancer of the invention; a developing section through which a reaction product of the sample and the detection agent migrates; a labeling section in which the developed reaction product is colored; and a display section in which the colored reaction product is developed. The test strip for immunochromatography can be in a form similar to that of a diagnostic agent for pregnancy. Upon application of a sample to the sample-receiving section, the sample-receiving section first absorbs the sample and then allows the sample to reach the reagent section. In the reagent section, subsequently, the colorectal cancer or esophageal cancer cell-derived cancer detecting marker in the sample reacts with the anti-COTL1 antibody, and the reaction complex migrates through the developing section to reach the labeling section. In the labeling section, the reaction complex reacts with a labeled secondary antibody. When the product of the reaction with the labeled secondary antibody is developed in the display section, a color is observed. The test strip for immunochromatography does not impose any pain or risk associated with use of reagents upon the user, and it can be used for at-home monitoring, the results of which can be thoroughly examined at medical institution for treatment (e.g., surgical resection), thus leading to the prevention of metastasis or recurrence. At present, this test strip can be mass-produced in a cost-effective manner by a production method as described in, for example, JP H10-54830 A (1988). In addition, the agent for detecting cancer of the present invention can be used in combination with an agent for detecting a known tumor marker for colorectal cancer or esophageal cancer to thereby realize diagnosis with higher reliability.

### 2-2. Step of determining suffering from cancer

In the "step of determining suffering cancer," whether or not the subject suffers from colorectal cancer or esophageal cancer is evaluated or determined on the basis of the amount of the protein measured in the step of measurement of the cancer detecting marker. Whether or not the subject suffers from colorectal cancer or esophageal cancer is determined on the basis of the measured mass of the cancer detecting marker, i.e., the COTL1 protein. For example, the subject is determined as having colorectal cancer or esophageal cancer when the amount of the cancer detecting marker measured in the subject is larger than that in a healthy individual, by a statistically significant degree.

The term "colorectal cancer" used herein refers to a malignant tumor developed in the large intestine (which is the caecum, the colon, and the rectum). Cecal cancer, colon cancer, and rectal cancer developed in such different regions are within the scope of "colorectal cancer." While colorectal cancer is pathologically classified as, for example, adenocarcinoma, endocrine cell carcinoma, adenosquamous carcinoma, or squamous carcinoma, colorectal cancer is not limited thereto.

The term "esophageal cancer" used herein refers to a malignant tumor developed in the esophagus (the alimentary tract from the throat to the stomach). While esophageal cancer is pathologically classified as, for example, squamous carcinoma, adenocarcinoma, or mucoepidermoid carcinoma, esophageal cancer is not limited thereto.

The term "healthy individual" used herein refers to an individual who is at least not afflicted with colorectal cancer or esophageal cancer, and preferably an individual who/which is healthy. The healthy individual is further required to be of the same species as the subject. When the subject to be examined is a human (i.e., a human subject), for example, the healthy individual must also be a human (hereinafter, referred to as a "healthy person"). It is preferable that a healthy individual has physical conditions that are the same as or similar to those of the subject. Examples of physical conditions of a human include race, sexuality, age, body height, and body weight.

An example of the use of the expression "statistically significant" is in a case in which the critical rate (i.e., the significance level) of the obtained value is less than 5% (p < 0.05), 1 %, or 0.1 %. Hence, the expression "statistically significantly larger" means that the statistical manipulation of the quantitative difference in the marker for detecting cancer obtained from the subject and the healthy individual, respectively, shows that there is a significant difference between the subject and the healthy individual and the amount of the protein in the subject is larger than that of the healthy individual. The expression "statistically significantly larger" generally refers to a situation in which the amount of the cancer detecting marker in the body fluid of the subject is larger than that of a healthy individual 2 or more times, preferably 3 or more times, 4 or more times, or 5 or more times, more preferably 10 or more times or 20 or more times, and further preferably 50 or more times. When the quantitative difference is 3 or more times, the reliability is high and thus such quantitative difference is considered statistically significant. A test method known in the art that allows determination of the presence or absence of significance can be used appropriately for testing the statistical manipulation without particular limitations. For example, a student's t test or a multiple comparison test can be used.

The amount of the cancer detecting marker in the body fluid of the healthy individual is preferably measured in the same manner as that used in the method for measuring the amount of the cancer detecting marker in the body fluid of the subject described in the preceding step. The amount of the cancer detecting marker in the body fluid of the healthy individual may be measured every time the amount of the cancer detecting marker in the body fluid of the subject is measured. Alternatively, the amount of the cancer detecting marker may be measured in advance. Particularly, the mass of the cancer detecting marker is measured in advance under various physical conditions of healthy individuals, and the values can be inputted to a computer so as to prepare a database. This approach is convenient because the amount of the cancer detecting marker derived from a healthy individual having physical conditions optimal for comparison with the subject can be immediately determined.

When the amount of the cancer detecting marker in the body fluid of the subject is statistically significantly larger than that in the body fluid of a healthy individual, the subject is diagnosed as having colorectal cancer or esophageal cancer. In the present invention, the disease stage of the target colorectal cancer or esophageal cancer may be any stage from early cancer to terminal cancer, without particular limitation.

As described above, the method for examining colorectal cancer or esophageal cancer of the present invention involves immunologically assaying the cancer detecting marker in a body fluid sample using an antibody. According to the method of the present invention, whether or not a subject has colorectal cancer or esophageal cancer can be determined or evaluated. In addition, patients who are afflicted with colorectal cancer or esophageal cancer can be distinguished from patients who are not suffering from colorectal cancer or esophageal cancer, respectively.

### 3. Kit for cancer diagnosis

The third aspect of the present invention relates to a kit for cancer diagnosis.

The "kit for cancer diagnosis" is directly or indirectly used for evaluation of affliction with cancer, the degree of disease, the occurrence of amelioration, or the degree of amelioration, occasionally for screening of a candidate substance useful in the prevention, amelioration, or treatment of cancer, and preferably for diagnosis of cancer.

The kit of the present invention encompasses, as a constituent, a substance capable of specifically recognizing and binding to the COTL1 protein, preferably the protein comprising the amino acid sequence as shown in SEQ ID NO: 1 or a variant sequence thereof, whose expression varies in a body fluid sample, and in particular, in blood, serum, or plasma, in relation to colorectal cancer or esophageal cancer affecting the subject. Specifically, the kit comprises, for example, the anti-COTL1 protein antibody, a fragment thereof, or a chemically modified derivative thereof. These antibodies may be conjugated to solid phase carriers of any configurations, such as wells, plates, or strips made of suitable materials (e.g., polymer or cellulose). Alternatively, the antibodies may be prepared in the form of test pieces for immunochromatography as described above. The kit may optionally contain, for example, a labeled secondary antibody and further, a substrate necessary for label detection, a carrier, a washing buffer, a sample diluent, an enzyme substrate, a reaction stop solution, a purified COTL1 protein serving as a standard, and instruction manuals.

### Examples

The present invention is described in greater detail with reference to the examples below, although the present invention is not limited to these examples.

### <Reference Example>

### (1) Preparation of hollow fiber filter

100 polysulfone hollow fibers with a molecular weight cutoff of 50,000 on the membrane surface were bundled, and both ends thereof were fixed to a glass tube using an epoxy potting agent while refraining from clogging the hollow portions of the hollow fibers. Thus, a minimodule was prepared. The minimodule (module A) is used for the removal of high-molecular-weight proteins in serum or plasma and has a diameter of approximately 7 mm and a length of approximately 17 cm. Likewise, a minimodule (module B) for use in concentration of low-molecular-weight proteins was prepared using a membrane with a molecular weight cutoff of approximately 3,000. Each minimodule has an inlet connected to the hollow fiber lumens at one end and an outlet at the other end. In a hollow fiber minimodule, the inlet is connected to the outlet through a silicon tube, so as to form a passage of a closed-circuit system in which a liquid is driven by Perista pump to circulate. Three modules A and one module B are connected in tandem via T-shaped connectors located in the middle of the passages to prepare a single hollow fiber filter. The glass tube serving as a jacket for the hollow fibers is equipped with a port for discharging a liquid that leaks out of the hollow fibers. Thus, a module set is constituted. This hollow fiber filter is washed with distilled water and filled with an aqueous solution of PBS (phosphate buffer containing 0.15 mM NaCl, pH 7.4). Serum or plasma used as a fractionation material is injected through the inlet into the passage of the hollow fiber filter and discharged from the outlet of the passage after fractionation and concentration. Each module A acts as a molecular sieve with a molecular weight cutoff of approximately 50,000 on the serum or plasma injected into the hollow fiber filter, and components having molecular weights smaller than 50,000 are concentrated in module B and thus are prepared.

### <Example 1>

### (1) Identification of protein in bloods of healthy persons and colorectal cancer patients

A mixed solution of sera obtained from 11 patients with colorectal cancer whose informed consent had been obtained and a mixed solution of sera obtained from 30 healthy persons of the same age cohort were prepared. Each mixed solution was filtered through a filter with a pore size of 0.22 µm to remove contaminants, and the protein concentration was adjusted to 50 mg/ml. This plasma was further diluted with a 25 mM ammonium bicarbonate solution (pH 8.0) to a concentration of 12.5 mg/ml and fractionated on the basis of molecular weight through the hollow fiber filter shown in Reference Example (1). The serum sample (total amount: 1.8 ml containing up to 250 µg of proteins) thus fractionated was freeze-dried and then redissolved in 100 µl of a 25 mM ammonium bicarbonate solution (pH 8.0). This sample was subjected to peptide digestion with trypsin in an amount that was 1/50 of the total protein amount under conditions of 37°C for 2 to 3 hours and desalting treatment with a desalting column (Waters Corp.), and then it was further fractionated into 8 fractions using an ion-exchange column (KYA Technologies Corp.). Each of the fractions was further fractionated using a reverse-phase column (KYA Technologies Corp.), and the eluted peptides were measured for their identifications three times using a Q-TOF Premier mass spectrometer (Micromass Ltd.) connected thereto online. The obtained data was analyzed using analysis software (Mascot; Matrix Science), and the proteins contained in the samples were identified. The results for the healthy controls and the cancer patients were compared, and, among the identified proteins, the COTL1 protein was found to be undetected in healthy controls Nos. 1 to 3 but detected in colorectal cancer patients Nos. 1 to 3. The scores for the reliability of identification concerning the COTL1 protein calculated at the time of analysis are shown in Table 1.

**Table 1**

| | Healthy person 1 | Healthy person 2 | Healthy person 3 | Colorectal cancer patient 1 | Colorectal cancer patient 2 | Colorectal cancer patient 3 |
|---|---|---|---|---|---|---|
| Score | 0 | 0 | 0 | 92 | 83 | 113 |

### (2) Detection of COTL1 protein in blood from colorectal cancer patient by Western blotting

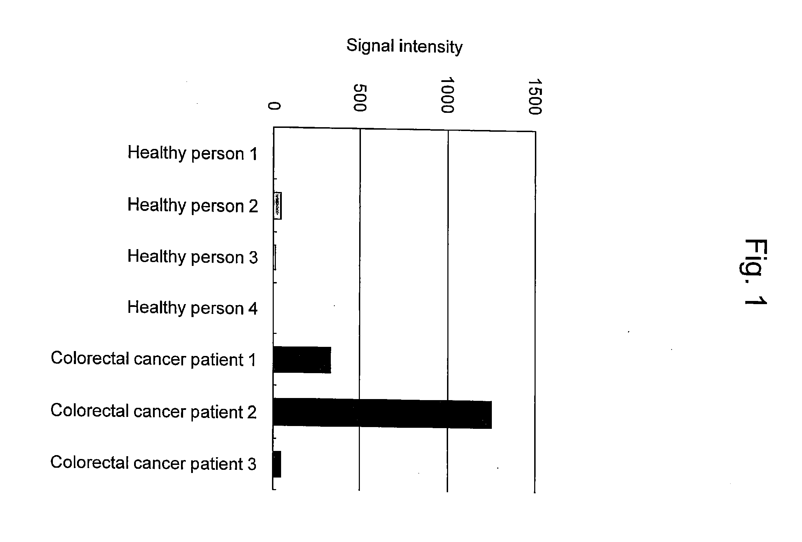

Plasma samples were obtained from 3 colorectal cancer patients and 4 healthy controls. Affi-Gel Blue (100 µl, Bio-Rad Laboratories, Inc.) and 50 µl of Protein A-Sepharose (GE Healthcare) were added to 100 µl of each sample, and the mixture was subjected to the reaction overnight at 4°C to remove albumin and immunoglobulin from the sample. The sample thus obtained was subjected to solubilization treatment with an SDS sample buffer (50 mM Tris-HCl, pH 6.8, 1 mM DTT, 5% SDS, 10% glycerol) and boiling treatment, the resultant was subjected to SDS-polyacrylamide gel (16%) electrophoresis, and proteins were then transferred to a PVDF membrane. This membrane was allowed to react with a rabbit anti-COTL1 polyclonal antibody (Proteintech Group Inc.) and further with a peroxidase-labeled secondary antibody (anti-rabbit IgG antibody). Proteins showing immune responses were visualized by exposure to an X-ray film using SuperSignal West Femto Maximum Sensitivity Substrate (Pierce Biotechnology, Inc.). The signal intensity of a band corresponding to COTL1 was digitalized by image analysis using ImageJ (NIH). As a result, the signal intensity was found to be higher in all 3 colorectal cancer patients than in the 4 healthy controls (Fig. 1).

The results of (1) and (2) demonstrate that the present invention is useful for detection of colorectal cancer.

### <Example 2>

### (1) Detection of COTL1 protein in blood from esophageal cancer patient by Western blotting

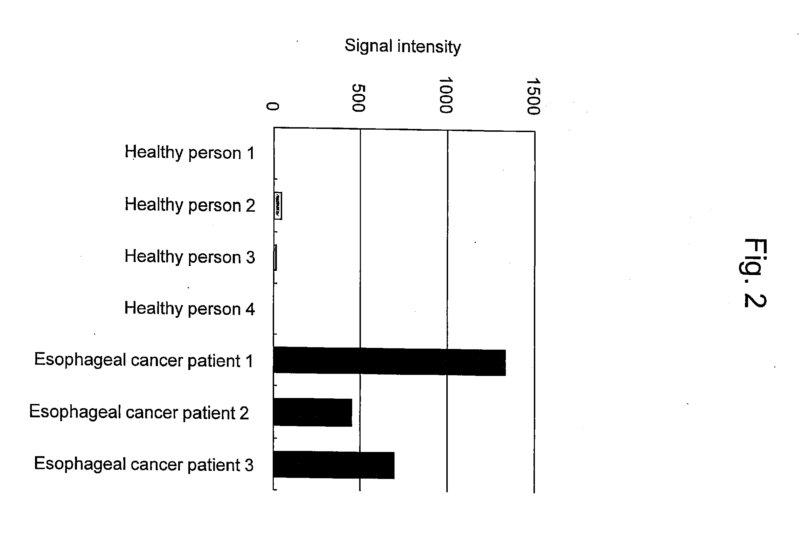

Plasma samples were obtained from 3 esophageal cancer patients, Nos. 1 to 3, whose informed consent had been obtained, and 4 healthy controls, Nos. 1 to 4. Affi-Gel Blue (100 µl, Bio-Rad Laboratories, Inc.) and 50 µl of Protein A-Sepharose (GE Healthcare) were added to 100 µl of each sample, and the mixture was subjected to the reaction overnight at 4°C to remove albumin and immunoglobulin from the sample. The sample thus obtained was subjected to solubilization treatment with an SDS sample buffer (50 mM Tris-HCl, pH 6.8, 1 mM DTT, 5% SDS, 10% glycerol) and boiling treatment, the resultant was subjected to SDS-polyacrylamide gel (16%) electrophoresis, and proteins were then transferred to a PVDF membrane. This membrane was allowed to react with a rabbit anti-COTL1 polyclonal antibody (Proteintech Group Inc.) and further with a peroxidase-labeled secondary antibody (anti-rabbit IgG antibody). Proteins showing immune responses were visualized by exposure to an X-ray film using SuperSignal West Femto Maximum Sensitivity Substrate (Pierce Biotechnology, Inc.). The signal intensity of a band corresponding to COTL1 was digitalized by image analysis using ImageJ (NIH). As a result, the signal intensity was found to be higher in all 3 esophageal cancer patients than in the 4 healthy controls (Fig. 2).

The results demonstrate that the present invention is useful for detection of esophageal cancer.

### Industrial Applicability

According to the present invention, colorectal cancer or esophageal cancer can be effectively detected in a simple and cost-effective manner that enables detection at an early stage, diagnosis, and treatment of colorectal cancer or esophageal cancer. According to the method of the present invention, further, colorectal cancer or esophageal cancer can be detected in a less invasive manner with the use of blood obtained from a patient. This enables detection of colorectal cancer or esophageal cancer in a simple and rapid manner.

All publications, patents, and patent applications cited herein are incorporated herein by reference in their entirety.

## Claims

1. A method for examining a cancer comprising measuring *in vitro* an amount of a cancer detecting marker consisting of a COTL1 protein present in a body fluid from a subject, and evaluating whether or not the subject suffers from a cancer selected from colorectal cancer and esophageal cancer on the basis of the amount of the marker.

2. The method according to claim 1, wherein the COTL1 protein is a polypeptide consisting of the amino acid sequence as shown in SEQ ID NO: 1, an amino acid sequence having 90% or higher identity with the amino acid sequence of SEQ ID NO: 1, or a partial sequence comprising at least 7-10 or more continuous amino acid residues constituting each of the amino acid sequences.

3. The method according to claim 1 or 2, wherein, when the amount of the cancer detecting marker in the subject is statistically significantly larger than that of a healthy individual, the subject is evaluated or determined as suffering a cancer selected from colorectal cancer and esophageal cancer.

4. The method according to claim 3, wherein the statistically significantly larger amount is two or more times that of a healthy individual.

5. The method according to any one of claims 1 to 4, wherein the measurement is performed using a substance capable of specifically binding to the cancer detecting marker.

6. The method according to claim 5, wherein the substance capable of binding is an anti-COTL1 antibody and/or a fragment thereof.

7. The method according to any one of claims 1 to 6, wherein the body fluid sample is blood or urine.

8. A kit for diagnosing a cancer selected from colorectal cancer and esophageal cancer, comprising an anti-COTL1 antibody, a fragment thereof, and/or a chemically modified derivative thereof.


SEQUENCE LISTING

<110>  TORAY INDUSTRIES, INC.
       KYOTO UNIVERSITY

<120>  Marker and method for detecting or examining colon cancer or
esophageal cancer

<130>  PH-5352-PCT

<150>  JP 2011-186147
<151>  2011-08-29

<160>  1

<170>  PatentIn version 3.5

<210>  1
<211>  142
<212>  PRT
<213>  Homo sapiens

<400>  1

Met Ala Thr Lys Ile Asp Lys Glu Ala Cys Arg Ala Ala Tyr Asn Leu
1               5                   10                  15

Val Arg Asp Asp Gly Ser Ala Val Ile Trp Val Thr Phe Lys Tyr Asp
            20                  25                  30

Gly Ser Thr Ile Val Pro Gly Glu Gln Gly Ala Glu Tyr Gln His Phe
        35                  40                  45

Ile Gln Gln Cys Thr Asp Asp Val Arg Leu Phe Ala Phe Val Arg Phe
    50                  55                  60

Thr Thr Gly Asp Ala Met Ser Lys Arg Ser Lys Phe Ala Leu Ile Thr
65                  70                  75                  80

Trp Ile Gly Glu Asn Val Ser Gly Leu Gln Arg Ala Lys Thr Gly Thr
                85                  90                  95

Asp Lys Thr Leu Val Lys Glu Val Val Gln Asn Phe Ala Lys Glu Phe
            100                 105                 110

Val Ile Ser Asp Arg Lys Glu Leu Glu Glu Asp Phe Ile Lys Ser Glu
        115                 120                 125

Leu Lys Lys Ala Gly Gly Ala Asn Tyr Asp Ala Gln Thr Glu
    130                 135                 140